# EUROPÄISCHE PATENTSCHRIFT

(11) **EP 4 003 448 B1**
(45) Veröffentlichungstag und Bekanntmachung des Hinweises auf die Patenterteilung: **25.12.2024**
(21) Anmeldenummer: 20745189.9
(22) Anmeldetag: 23.07.2020
(51) Int. Cl.: A61M 1/16

(54) **VORRICHTUNG ZUR BEREITSTELLUNG VON DIALYSEKONZENTRAT**
DEVICE FOR PROVIDING DIALYSIS CONCENTRATE
DISPOSITIF DESTINÉ À FOURNIR UN CONCENTRÉ POUR DIALYSE

(30) Priorität: 25.07.2019 DE 102019120174
(43) Veröffentlichungstag der Anmeldung: 01.06.2022
(73) Patentinhaber: Fresenius Medical Care GmbH, 61352 Bad Homburg (DE)
(72) Erfinder: ENGELN, Florian, 35321 Laubach (DE)
(74) Vertreter: Herrmann, Uwe
(86) Internationale Anmeldenummer: PCT/EP2020/070832
(87) Internationale Veröffentlichungsnummer: WO 2021/013945

(56) Entgegenhaltungen:
- EP-A1- 0 619 135
- EP-A1- 2 896 417
- EP-A2- 2 745 862
- DE-A1- 102017 218 209

## Beschreibung

Die vorliegende Erfindung betrifft eine Vorrichtung zur Bereitstellung von flüssigem Dialysekonzentrat umfassend einen Dialysekonzentratbehälter zur Aufnahme des Dialysekonzentrats.

Aus dem Stand der Technik ist es bekannt, flüssiges Dialysekonzentrat in einem oder mehreren Dialysekonzentratbehältern vorzuhalten, um aus den Dialysekonzentraten durch entsprechende Verdünnung, üblicherweise mit RO-Wasser, die gebrauchsfertige Dialyselösung herzustellen.

Zur Herstellung der Dialysekonzentrate werden üblicherweise in einem Wechselbehälter Trockenkonzentrate oder aufgeschlämmte Konzentrate vorgelegt. Nach der Konnektion des Wechselbehälters an die Vorrichtung wird in den Wechselbehälter Wasser gepumpt und die Mischung in einem Rezirkulationskreislauf durch einen Mischtank und den Wechselbehälter solange rezirkuliert, bis alle Feststoffe des Trockenkonzentrats aufgelöst sind. Ist dies der Fall, wird das auf diese Weise hergestellte flüssige Dialysekonzentrat in einen oder mehrere Dialysekonzentratbehälter gefördert und von dort beispielsweise einer Zentralversorgungseinheit für Dialysekonzentrate zugeführt.

Ein wesentlicher Aspekt bei dieser Vorgehensweise ist die vollständige Auflösung des Trockenkonzentrats, um die gewünschte Zusammensetzung des flüssigen Dialysekonzentrats zu gewährleisten. Um dies prüfen zu können, werden zwei Varianten angewandt: zum einen die Entnahme einer Probe des Dialysekonzentrats und deren Versendung an ein Labor und zum anderen die Dichtemessung durch den Betreiber selbst mittels eines Handdichtemessgerätes.

Beide Varianten sind aufwändig, so dass der vorliegenden Erfindung die Aufgabe zugrunde liegt, eine Vorrichtung der eingangs genannten Art dahingehend weiterzubilden, dass die Dichtebestimmung für den Betreiber, d.h. für den Nutzer der Vorrichtung besonders einfach und zeitsparend möglich ist.

Weiterhin ist es aus dem Stand der Technik, beispielsweise aus der Druckschrift EP 2745862 A1 bekannt, eine Mischanlage zur Herstellung gebrauchsfähiger Lösungen aus Konzentraten und hochreinem Wasser für den Einsatz als Konzentrat oder Spüllösung für die Hämodialyse mit einer integrierten Messvorrichtung zur Messung der Dichte der erzeugten Lösungen auszustatten.

Die aus der EP 2745862 A1 bekannte Mischanlage weist einen Tank auf, in welchen Lösungsmittel, z.B. Wasser, dosiert wird. Anschließend wird das Lösungsmittel in einen Tank mit Trockenkonzentrat gefördert, woraufhin das Trockenkonzentrat durch eine Zirkulation der Suspension durch den Tank, den Rohstoffbehälter und die daran angeschlossenen Leitungen, welche gemeinsam einen Zirkulationskreislauf bilden, in Lösung gebracht wird.

Die Messvorrichtung zur Dichtemessung ist hierbei in dem Zirkulationskreislauf angeordnet, beispielsweise kann hierfür eine Messkammer vorgesehen sein. Die Messvorrichtung zur Dichtemessung ist somit ein Bestandteil der Mischanlage, insbesondere deren Zirkulationskreislauf, und wird von dem Gemisch aus Trockenkonzentrat und Lösungsmittel umströmt.

Derartige Mischanlagen sind jedoch recht teuer und die meisten bestehenden Aufbereitungsanlagen weisen keine derartige integrierte Dichtemessung auf, weswegen die Dichte der erzeugten Lösungen in der Praxis meist händisch gemessen werden muss.

Eine weitere Aufgabe der vorliegenden Erfindung besteht somit darin, eine Möglichkeit zu schaffen, bestehende Aufbereitungsanlagen für eine einfache und zeitsparende Dichtemessung durch den Benutzer nachzurüsten.

Weiterhin betrifft die DE102017218209A1 eine Vorrichtung zur Entgasung von Dialysekonzentraten für die automatische Dichtemessung in Mischanlage. Die EP0619135A1 offenbart ein System zur Herstellung von Flüssigkeit für eine medizinische Behandlung.

Allgemein formuliert besteht die Aufgabe der vorliegenden Erfindung somit darin, die Probleme des Stands der Technik abzumildern oder gar ganz zu beseitigen.

Diese Aufgabe wird durch eine Vorrichtung mit den Merkmalen des Anspruchs 1 gelöst.

Die Erfindung wird durch die Merkmale des unabhängigen Anspruchs 1 definiert.

Danach ist vorgesehen, dass der Dialysekonzentratbehälter einen Ablauf aufweist und dass die Vorrichtung eine Messeinrichtung aufweist, die mit dem Ablauf in Fluidverbindung steht und die ausgebildet ist, die Dichte oder eine mit der Dichte korrelierte Eigenschaft des Dialysekonzentrats zu ermitteln, wobei in dem Ablauf ein Absperrelement, insbesondere ein Ventil angeordnet ist, mittels dessen der Ablauf selektiv geöffnet oder verschlossen werden kann.

Hierbei ist der Dialysekonzentratbehälter vorzugsweise kein Teil eines Zirkulationskreislaufs und kommt nicht bei der Auflösung eines Konzentrats in einem Lösungsmittel, z.B. Wasser, zum Einsatz. In anderen Worten wird vorzugsweise der Dialysekonzentratbehälter nicht beim Mischen eines Konzentrats und eines Lösungsmittels von dem Gemisch aus Trockenkonzentrat und dem Lösungsmittel durchströmt.

In anderen Worten ist vorzugsweise der Dialysekonzentratbehälter somit separat und / oder unabhängig von einem Zirkulationskreislauf einer Aufbereitungsanlage oder Mischanlage ausgebildet und wird nicht von einem zirkulierenden Gemisch aus Konzentrat und Lösungsmittel durchströmt.

Erfindungsgemäß weist der Zirkulationskreislauf einen Mischbehälter und einen Wechselbehälter auf, welche miteinander über eine (Re-)zirkulationsleitung verbunden sind. Das fertig gemischte Dialysekonzentrat wird vorzugsweise nach der Rezirkulation in den Dialysekonzentratbehälter geleitet.

Der Ablauf kann eine Leitung, insbesondere einen Schlauch oder ein Rohr umfassen, mittels dessen das Dialysekonzentrat aus dem Dialysekonzentratbehälter zu der Messeinrichtung geleitet wird. Wird eine Dichtemessung oder die Messung einer Eigenschaft gewünscht, mittels derer auf die Dichte rückgeschlossen werden kann (wie z.B. die Lichtdurchlässigkeit etc.), wird das Absperrelement geöffnet, so dass das Dialysekonzentrat von dem Dialysekonzentratbehälter zu der Messeinrichtung geleitet wird.

Der Ablauf ist somit vorzugsweise nicht dazu ausgelegt, mit einer Aufbereitungsanlage und / oder einem Zirkulationskreislauf einer Aufbereitungsanlage verbunden zu werden, sondern dient vorzugsweise ausschließlich der Entleerung des Dialysekonzentratbehälters. Die vorliegende Erfindung ist somit auf jeglichen Dialysekonzentrabehälter anwendbar.

Dieses Öffnen des Absperrelements kann manuell bei Bedarf durch den Nutzer erfolgen oder auch automatisiert durch eine Steuereinheit, die das Öffnen des Absperrelementes veranlasst.

Wenn im Folgenden von einer Dichtemessung die Rede ist, umfasst dies nicht nur die Messung der Dichte des Dialysekonzentrats, sondern schließt dies die Messung jeder beliebigen Eigenschaft mit ein, mit der auf die Dichte des Dialysekonzentrats rückgeschlossen werden kann.

Exemplarisch sind als mit der Dichte korrelierte Eigenschaft die Lichtabsorption des Dialysekonzentrats, die Transmission von Licht durch das Dialysekonzentrat, die Konzentration eines, mehrerer oder aller Inhaltsstoffe des Dialysekonzentrats, die Leitfähigkeit des Dialysekonzentrats, das Vorhandensein nicht gelöster Feststoffe im Dialysekonzentrat oder die Viskosität des Dialysekonzentrats oder eine Kombination der vorgenannten Parameter zu nennen.

In dem Ablauf kann ein Rückflussverhinderungselement angeordnet sein, wie z.B. eine Rückschlagklappe.

Erfindungsgemäß sind mehrere Dialysekonzentratbehälter vorhanden, die jeweils über einen Ablauf verfügen, wobei die mehreren Abläufe in einem Verteiler zusammengeführt sind, der in Flussrichtung des Dialysekonzentrats stromaufwärts der Messeinrichtung angeordnet ist. In diesem Fall ist vorzugsweise jeder Dialysekonzentratbehälter mit einem Absperrelement, wie z.B. mit einem Probenahmehahn ausgestattet. An diesen Absperrelementen sind Konzentratschläuche angeschlossen, die in einem Verteilerblock oder dergleichen zusammengeführt sind, aber fluidisch voneinander getrennt sind.

Pro Ablauf können ein Absperrelement oder auch mehrere Absperrelemente vorgesehen sein.

Das oder die Absperrelemente können an oder in dem Verteiler angeordnet sein. So ist es beispielsweise denkbar, an dem oder den Dialysekonzentratbehältern je einen Probenahmehahn oder dergleichen anzuordnen und vor dem Eintritt in den Verteiler je einen Kugelhahn oder dergleichen und ggf. ein Rückflussverhinderer.

Vorzugsweise ist weiterhin vorgesehen, dass vom Verteiler wegführend ein Konzentratschlauch oder eine sonstige Konzentratleitung an einem Inline-Dichtemessgerät, d.h. an der erfindungsgemäßen Messeinrichtung angeschlossen ist.

Denkbar ist es, dass zur Ermittlung der Dichte des in dem Dialysekonzentratbehälter befindlichen Dialysekonzentrats bei geöffnetem Probenahmehahn am Verteiler der entsprechende Kugelhahn geöffnet wird, so dass das Konzentrat aus dem betreffenden Dialysekonzentratbehälter durch das Dichtemessgerät fließt und von diesem vorzugsweise in den Abfluss.

Bevorzugt ist es wenn, der Ablauf ohne Freifallstrecke in seinem Endbereich in eine Flüssigkeit, z.B. in einem Siphon ragt, so dass das System luftfrei ist. Das gesamte System ist somit permanent luftfrei, interne mögliche Auskristallisierungen und Verblockungen des Konzentrats können so vermieden werden.

In einer weiteren Ausgestaltung der Erfindung ist vorgesehen, dass die Vorrichtung ein Lesegerät oder ein Eingabegerät aufweist, mittels dessen der Konzentrattyp einlesbar bzw. eingebbar ist, aus dem das Dialysekonzentrat hergestellt wird und dass eine Datenbank oder ein sonstiger Speicher vorhanden ist, in der eine Korrelation zwischen dem Konzentrattyp und der Dichte oder der damit korrelierten Eigenschaft des Dialysekonzentrats gespeichert ist.

Mittels des Lesegerätes wird der Konzentrattyp des Konzentrats, vorzugsweise des Trockenkonzentrats ermittelt, das sich in einem entsprechenden Konzentratbehälter befindet, der im Folgenden auch als Wechselbehälter bezeichnet wird. Bei dem Lesegerät handelt es sich beispielsweise um ein Barcode-Hand-Lesegerät oder um einen sonstigen Scanner.

Dieses/dieser ist an einem PC oder dergleichen angeschlossen, wie auch die Messeinrichtung, vorzugsweise das Dichtemessgerät, und ggf. ein Etikettendrucker und/oder eine Signalisierungseinrichtung, mittels derer dem Nutzer signalisiert werden kann, ob das Dialysekonzentrat die korrekte Dichte aufweist und somit eine vollständige Auflösung des Trockenkonzentrats stattgefunden hat.

Dazu kann eine Vergleichseinheit vorhanden sein, die die gemessene Dichte/Eigenschaft mit der in der Datenbank gespeicherten Dichte/Eigenschaft vergleicht.

Die Vergleichseinheit kann ausgebildet sein, ein von dem Vergleich abhängiges, für den Nutzer wahrnehmbares Signal auszugeben und/oder ein Etikett auszudrucken, auf dem das Dialysekonzentrat betreffende Daten aufgedruckt sind. Letzteres findet vorzugsweise nur dann statt, wenn die gemessene Dichte bzw. mit dieser korrelierte Eigenschaft in einem Sollwertbereich liegt.

Um sicherzustellen, dass die Messeinrichtung die Dichte oder dergleichen des korrekten Dialysekonzentrats und nicht Rückstände einer vorausgegangenen Messung misst, ist es bevorzugt, wenn eine Steuerung vorhanden ist, die die Messeinrichtung erst nach Ablauf einer bestimmten Zeitspanne nach der Öffnung des Absperrelementes oder nach einer sonstigen Aktion aktiviert. So ist es beispielsweise denkbar, dass die Steuerung erst 30 Sekunden oder dergleichen nach dem Öffnen des Kugelhahns oder nach einem sonstigen Triggerereignis die Dichtemessung vornimmt.

Denkbar ist es, dass die Steuerung so ausgebildet ist, dass die Messeinrichtung erst eine bestimmte Zeitspanne nach Aktivierung des Lesegerätes und/oder des Eingabegerätes aktiviert wird und die Messung der Dichte oder der sonstigen Eigenschaft des flüssigen Dialysekonzentrats vornimmt.

Erfindungsgemäß weist die Vorrichtung einen Mischbehälter sowie einen Wechselbehälter auf, in dem das Konzentrat enthalten ist, aus dem das Dialysekonzentrat hergestellt wird. Der Wechselbehälter dient zur Aufnahme des vorzugsweise als Trockenkonzentrat vorliegenden Konzentrats, aus dem das flüssige Dialysekonzentrat hergestellt wird.

An dieser Stelle wird darauf hingewiesen, dass die Erfindung sowohl den Fall umfasst, dass die Behälter, Tanks etc. gefüllt sind als auch den Fall, dass dies nicht der Fall ist. Durch die vorliegende Erfindung wird somit die Vorrichtung als solche als auch die Vorrichtung im befüllten Zustand geschützt.

Im befüllten Zustand befindet sich in dem Wechselbehälter das Konzentrat (vorzugsweise Trockenkonzentrat), aus dem das flüssige Dialysekonzentrat hergestellt wird und in dem Rezirkulationskreislauf, der den Mischbehälter, eine Pumpe sowie den Wechselbehälter mit einschließt, erfolgt die Mischung bzw. Lösung dieses Konzentrats mit Wasser. In dem Dialysekonzentratbehälter befindet sich das flüssige Dialysekonzentrat, aus dem dann in einem weiteren Schritt die gebrauchsfertige Dialyselösung hergestellt wird.

Erfindungsgemäß stehen der Mischbehälter und der Wechselbehälter durch eine Rezirkulationsleitung miteinander in Fluidverbindung.

Erfindungsgemäß ist weiterhin vorgesehen, dass der Mischbehälter mit dem oder den Dialysekonzentratbehältern in Fluidverbindung steht, um das Dialysekonzentrat von dem Mischbehälter mittels einer Pumpe in den oder die Dialysekonzentratbehälter zu überführen.

Erfindungsgemäß ist vorgesehen, dass mehrere Dialysekonzentratbehälter vorhanden sind, in denen Dialysekonzentrate mit jeweils unterschiedlicher Zusammensetzung enthalten sind.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Einzelheiten und Vorteile der Erfindung werden anhand eines in der Zeichnung dargestellten Ausführungsbeispiels näher erläutert.

Es zeigen:
- Figur 1:: eine schematische Ansicht der erfindungsgemäßen Vorrichtung mit mehreren Dialysekonzentratbehältern,
- Figur 2:: eine schematische Darstellung des Verteilerblocks mit Messgerät, PC, Scanner und Etikettendrucker,
- Figur 3:: eine schematische Darstellung des Verteilerblocks mit Messgerät, Tablet und Etikettendrucker und
- Figur 4:: eine exemplarische Darstellung des Etikettendruckers sowie eines Etiketts für das Konzentratprotokoll.

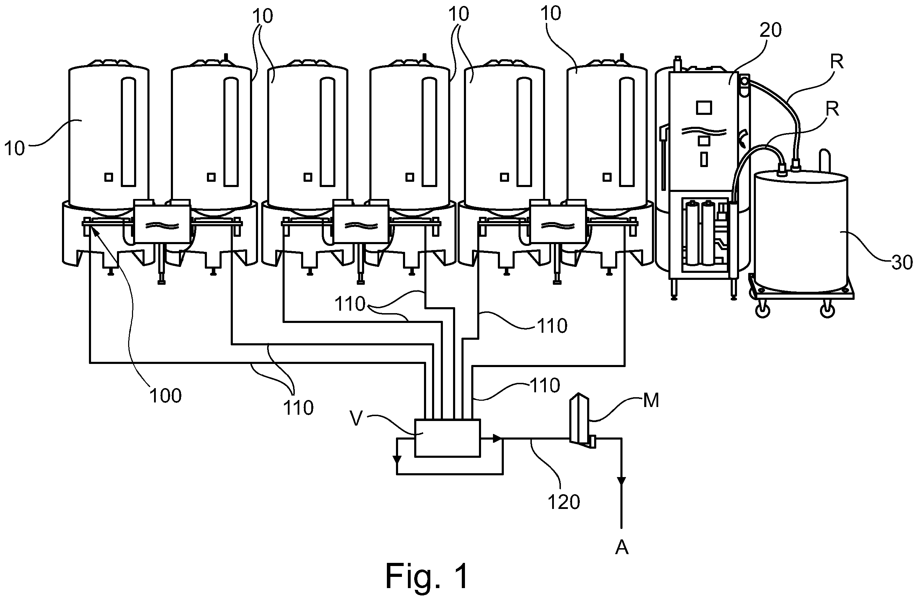

Figur 1 zeigt eine Vorrichtung gemäß der Erfindung mit insgesamt sechs Dialysekonzentratbehältern 10, in denen sich das flüssige Dialysekonzentrat befindet. Des Weiteren ist ein Mischbehälter 20 vorgesehen, der über Rezirkulationsleitungen R mit einem Wechselbehälter 30 verbunden ist.

In dem Wechselbehälter 30 befindet sich ein Trockenkonzentrat oder ein aufgeschlämmtes Konzentrat, das mit Wasser, vorzugsweise RO-Wasser, aus dem Mischbehälter 20 vermischt wird und durch Rezirkulation zwischen Wechselbehälter 30 und Mischbehälter 20 aufgelöst wird. Die Rezirkulation wird durch eine nicht näher dargestellte Pumpe bewirkt.

Nach der Auflösung des Trockenkonzentrats bzw. des aufgeschlämmten Konzentrats gelangt das Dialysekonzentrat mittels einer nicht näher dargestellten Pumpe in die Dialysekonzentratbehälter 10, wobei in allen Dialysekonzentratbehältern dasselbe Dialysekonzentrat oder auch unterschiedliche Dialysekonzentrate vorliegen können.

Jeder Dialysekonzentratbehälter 10 ist mit einem Probenahme-Hahn 100 ausgestattet, wobei dieser der besseren Übersichtlichkeit halber nur bei dem links dargestellten Dialysekonzentratbehälter 10 mit einem Bezugszeichen versehen ist.

Die Probenahme-Hähne 100 werden zum Zweck der Entnahme von Konzentrat-Proben verwendet. Es sind an allen Probenahme-Hähnen 100 (hier sechs Stück) Konzentratschläuche 110 angeschlossen. Diese werden an einem Verteiler-Block V zusammengeführt, wie dies aus Figur 1 hervorgeht.

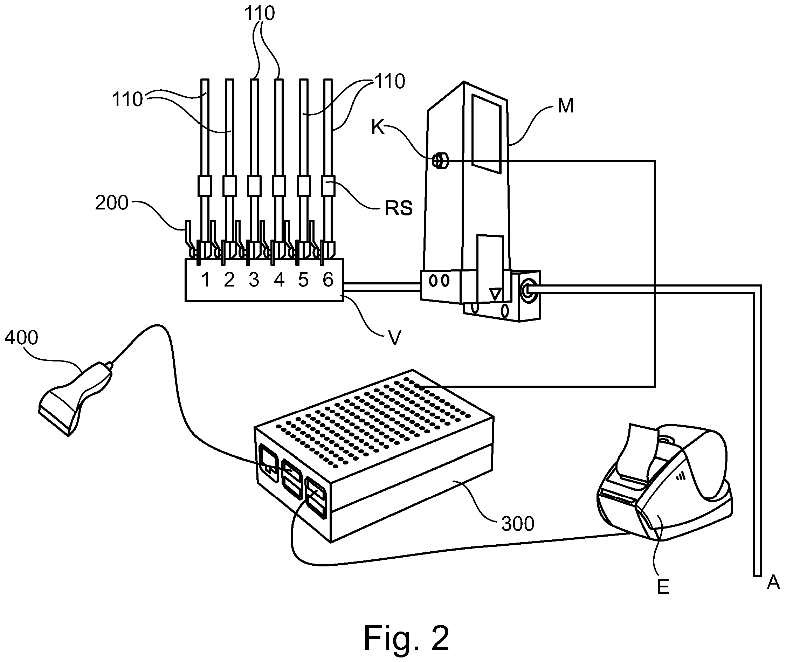

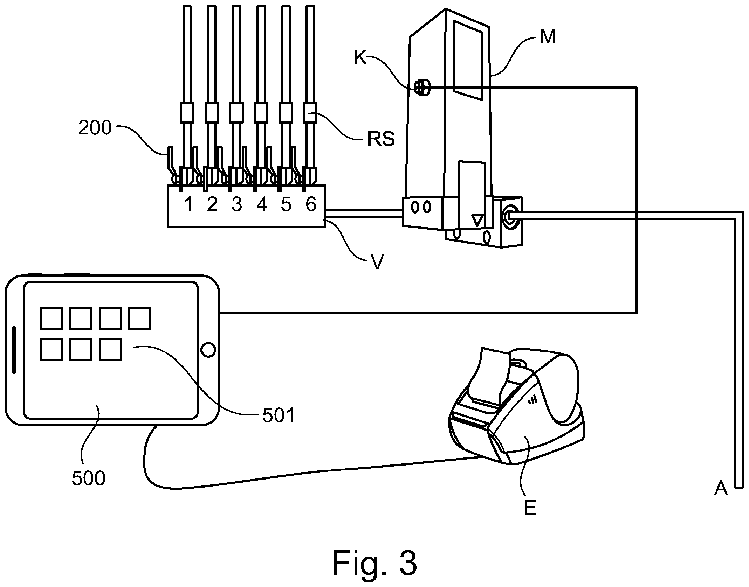

Vor Eintritt in den Verteilerblock V befinden sich je ein Kugelhahn 200 und je ein Rückflussverhinderer RS, wie dies aus den Figuren 2 und 3 hervorgeht. Aus diesen Figuren geht auch hervor, dass jeder Schlauch 110 über einen eigenen Anschluss an dem Verteiler V angeschlossen ist.

Vom Verteilerblock V wegführend ist ein Konzentratschlauch 120 an einem Inline-Dichtemessgerät M angeschlossen, wie dies in den Figuren 1 bis 3 dargestellt ist.

Zur Ermittlung der Dichte des in im Dialysekonzentratbehälter 10 befindlichen flüssigen Dialysekonzentrats wird am Verteiler-Block V der entsprechende Kugelhahn 200 geöffnet. Das Dialysekonzentrat fließt somit durch das Dichtemessgerät M und von diesem in den Abfluss A.

Das Ende des Abflussschlauches 120 ragt ohne Freifallstrecke in eine Flüssigkeit z.B. in einen Siphon hinein. Das gesamte Konzentrat-Probenahme-System ist somit permanent luftfrei. Interne mögliche Auskristallisierungen und Verblockungen des Konzentrats können so vermieden werden.

Figur 2 zeigt mit dem Bezugszeichen 300 einen PC, vorzugsweise einen Mini-PC mit spezieller Software, in der alle (z.B. sieben) Konzentrattypen mit zugehörigen Dichtegrenzen und Temperatur-Korrekturwerten hinterlegt sind. An dem PC 300 ist folgende Hardware angeschlossen: über einen RS 232 Konnektor K das Dichtemessgerät M, ein Barcode Hand Lesegerät 400 und ein (Mini) Etikettendrucker E.

Nach Öffnen des Kugelhahns 200 strömt das Dialysekonzentrat aus dem zu beprobenden Dialysekonzentratbehälter 10 beispielsweise mit 200 ml/min durch das Dichtemessgerät M in den Abfluss A.

Nachdem der Nutzer mit dem Barcode Lesegerät 400 das Label am Wechselbehälter 30 gescannt hat, zählt die Software 30 Sekunden (oder eine sonstige geeignete Zeitspanne) bis zur Übernahme, d.h. Messung des aktuellen Dichte-und Temperaturwertes herunter. Nach diesen 30 Sekunden ist gewährleistet, dass sämtliche von der letzten Messung verbliebenen Füllvolumina das System verlassen haben.

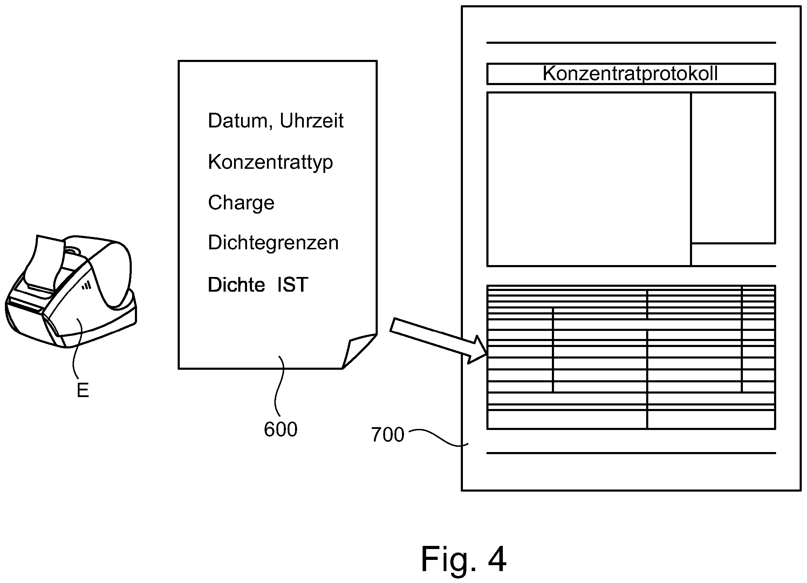

Liegt der gemessene Dichtewert innerhalb der Toleranzgrenzen, erfolgt ein entsprechender Etiketten-Ausdruck, wie er in Figur 4 gezeigt ist. Auf dem Etikett 600 sind Datum, Uhrzeit der Messung, der Konzentrattyp, die Charge, die Dichtegrenzen und der gemessene Wert der Dichte angegeben. Das Bezugszeichen 700 in Figur 4 zeigt ein Konzentratprotokoll.

Anstelle eines Lesegerätes kann auch ein Tablet verwendet werden, wie dies in Figur 3 gezeigt ist. Auf dem Tablet 500 befindet sich eine App mit einer Auswahl des Konzentrattyps, in der alle (z.B. sieben) Konzentrattypen mit zugehörigen Dichtegrenzen und Temperatur-Korrekturwerten hinterlegt sind. An dem Tablet 500 ist folgende Hardware angeschlossen: Das Dichtemessgerät M sowie der (Mini) Etikettendrucker E.

Wie auch bei der zu Figur 2 beschriebenen Vorgehensweise strömt nach dem Öffnen des Kugelhahns 200 das Dialysekonzentrat aus dem zu beprobenden Dialysekonzentratbehälter 10 mit z.B. 200 ml/min durch das Dichtemessgerät M in den Abfluss A.

Nachdem der Anwender am Display des Tablets 500 den verwendeten Konzentrattyp aus der Auswahl 501 angewählt hat, zählt die Software 30 Sekunden bis zur Übernahme, d.h. Messung des aktuellen Dichte-und Temperaturwertes herunter.

Nach diesen 30 Sekunden ist gewährleistet, dass sämtliche von der letzten Messung verbliebenen Füllvolumina das System verlassen haben. Liegt der Dichtewert innerhalb der Toleranzgrenzen, erfolgt ein entsprechender Etiketten-Ausdruck, wie dies bereits zu Figur 4 beschrieben wurde.

Die erfindungsgemäße Messanordnung kann von vornherein an der Vorrichtung angeordnet werden oder als Nachrüstsatz an bestehende Vorrichtungen zur Herstellung eines flüssigen Dialysekonzentrats montiert werden. Dazu müssen die Dialysekonzentratbehälter mit einem Ablauf versehen werden sowie das beschriebene Messequipment bereitgestellt werden.

## Patentansprüche

1. Vorrichtung zur Bereitstellung von flüssigem Dialysekonzentrat umfassend einen Dialysekonzentratbehälter (10) zur Aufnahme des Dialysekonzentrats, wobei der Dialysekonzentratbehälter (10) einen Ablauf aufweist und dass die Vorrichtung eine Messeinrichtung (M) aufweist, die mit dem Ablauf in Fluidverbindung steht und die ausgebildet ist, die Dichte des Dialysekonzentrats zu ermitteln, wobei in dem Ablauf ein Absperrelement angeordnet ist, mittels dessen der Ablauf selektiv geöffnet oder verschlossen werden kann, wobei mehrere Dialysekonzentratbehälter (10) vorhanden sind, in denen Dialysekonzentrate mit jeweils unterschiedlicher Zusammensetzung enthalten sind und die jeweils über einen Ablauf verfügen, wobei die mehreren Abläufe in einem Verteiler (V) zusammengeführt sind, in dem mehrere Absperrelemente angeordnet sind und der in Flussrichtung des Dialysekonzentrats stromaufwärts der Messeinrichtung (M) angeordnet ist, wobei die Vorrichtung einen Mischbehälter (20) sowie einen Wechselbehälter (30) aufweist, in dem Trockenkonzentrat enthalten ist, aus dem das Dialysekonzentrat hergestellt wird und der Mischbehälter (20) und der Wechselbehälter (30) durch eine Rezirkulationsleitung miteinander in Fluidverbindung stehen und dass der Mischbehälter (20) mit den Dialysekonzentratbehältern (10) in Fluidverbindung steht, um das Dialysekonzentrat von dem Mischbehälter (20) in den oder die Dialysekonzentratbehälter (10) zu überführen.

2. Vorrichtung nach Anspruch 1, **dadurch gekennzeichnet, dass** in dem Ablauf ein Rückflussverhinderungselement angeordnet ist und/oder dass an dem Dialysekonzentratbehälter (10) und/oder stromabwärts des Dialysekonzentratbehälters im Ablauf ein Probenahmehahn (100) als Absperrelement angeordnet ist.

3. Vorrichtung nach einem der vorhergehenden Ansprüche, **dadurch gekennzeichnet, dass** der Ablauf ohne Freifallstrecke in seinem Endbereich in eine Flüssigkeit ragt, so dass das System luftfrei ist.

4. Vorrichtung nach einem der vorhergehenden Ansprüche, **dadurch gekennzeichnet, dass** die Vorrichtung ein Lesegerät (400) oder ein Eingabegerät aufweist, mittels dessen der Konzentrattyp einlesbar bzw. eingebbar ist, aus dem das Dialysekonzentrat hergestellt wird und dass ein Speicher vorhanden ist, in der eine Korrelation zwischen dem Konzentrattyp und der Dichte oder der damit korrelierten Eigenschaft des Dialysekonzentrats gespeichert ist.

5. Vorrichtung nach Anspruch 4, **dadurch gekennzeichnet, dass** eine Vergleichseinheit vorhanden ist, die ausgebildet ist, die gemessene Dichte bzw. die mit dieser korrelierte Eigenschaft mit der in dem Speicher gespeicherten Dichte bzw. Eigenschaft zu vergleichen.

6. Vorrichtung nach Anspruch 5, **dadurch gekennzeichnet, dass** die Vergleichseinheit ausgebildet ist, ein von dem Vergleich abhängiges, für den Nutzer wahrnehmbares Signal auszugeben und/oder den Ausdruck eines Etiketts zu veranlassen, auf dem das Dialysekonzentrat betreffende Daten aufgedruckt sind.

7. Vorrichtung nach einem der vorhergehenden Ansprüche, **dadurch gekennzeichnet, dass** eine Steuerung vorhanden ist, die die Messeinrichtung (M) erst nach Ablauf einer bestimmten Zeitspanne nach der Öffnung des Absperrelementes aktiviert.

8. Vorrichtung nach einem der Ansprüche 4 bis 6, **dadurch gekennzeichnet, dass** eine Steuerung vorhanden ist, die die Messeinrichtung erst nach Aktivierung bzw. Betätigung des Lesegerätes (400) und/oder des Eingabegerätes aktiviert.

## Claims

1. An apparatus for providing liquid dialysis concentrate comprising a dialysis concentrate container (10) for receiving the dialysis concentrate, wherein the dialysis concentrate container (10) has an outflow; wherein the apparatus has a measurement device (M) that is in fluid communication with the outflow and that is configured to determine the density of the dialysis concentrate; wherein a shut-off element is arranged in the outflow by means of which the outflow can be selectively opened or closed; wherein a plurality of dialysis concentrate containers (10) are present in which dialysis concentrates each having different compositions are contained and that each have an outflow, with the plurality of outflows being brought together in a manifold (V) in which a plurality of shut-off elements are arranged and that is arranged upstream of the measurement device (M) in the direction of flow of the dialysis concentrate; wherein the apparatus has a mixing container (20) and a swap container (30) in which dry concentrate, from which the dialysis concentrate is prepared, is contained ; wherein the mixing container (20) and the swap container (30) are in fluid communication with one another by a recirculation line; and wherein the mixing container (20) is in fluid communication with the dialysis concentrate containers (10) to transfer the dialysis concentrate from the mixing container (20) into the dialysis concentrate container or containers (10).

2. An apparatus in accordance with claim 1, **characterized in that** a back flow prevention element is arranged in the outflow; and/or **in that** a sampling faucet (100) is arranged at the dialysis concentrate container (10) and/or in the outflow downstream of the dialysis concentrate container as a shut-off element.

3. An apparatus in accordance with one of the preceding claims, **characterized in that** the outflow projects into a liquid without a gravity path in its end region so that the system is free of air.

4. An apparatus in accordance with one of the preceding claims, **characterized in that** the apparatus has a reading device (400) or an input device by means of which the concentrate type from which the dialysis concentrate is prepared can be read in or input; and **in that** a store is present in which a correlation between the concentrate type and the density or the property correlated therewith of the dialysis concentrate is stored.

5. An apparatus in accordance with claim 4, **characterized in that** a comparison unit is present that is configured to compare the measured density or the property correlated therewith with the density or property stored in the store.

6. An apparatus in accordance with claim 5, **characterized in that** the comparison unit is configured to output a signal dependent on the comparison and perceptible to the user and/or to initiate the printing of a label on which the data relating to the dialysis concentrate are printed.

7. An apparatus in accordance with one of the preceding claims, **characterized in that** a control is present that only activates the measurement device (M) after expiration of a specific time period after the opening of the shut-off element.

8. An apparatus in accordance with one of the claims 4 to 6, **characterized in that** a control is present that only activates the measurement device after activation or actuation of the reading device (400) and/or of the input device.

## Revendications

1. Dispositif destiné à fournir un concentré liquide pour dialyse comprenant un récipient de concentré pour dialyse (10) destiné à recevoir le concentré pour dialyse, le récipient de concentré pour dialyse (10) présentant une évacuation et le dispositif présentant un moyen de mesure (M) qui est en liaison fluidique avec l'évacuation et qui est conçu pour déterminer la masse volumique du concentré pour dialyse, un élément d'arrêt étant agencé dans l'évacuation, au moyen duquel l'évacuation peut être sélectivement ouverte ou fermée, dans lequel
plusieurs récipients de concentré pour dialyse (10) sont prévus, dans lesquels se trouvent des concentrés pour dialyse dotés chacun d'une composition différente et qui disposent chacun d'une évacuation, les plusieurs évacuations étant réunies dans un collecteur (V) dans lequel plusieurs éléments d'arrêt sont agencés et qui est agencé, dans la direction d'écoulement du concentré pour dialyse, en amont du moyen de mesure (M), dans lequel
le dispositif présente un récipient de mélange (20) ainsi qu'un récipient interchangeable (30) dans lequel se trouve du concentré sec à partir duquel le concentré pour dialyse est fabriqué et le récipient de mélange (20) et le récipient interchangeable (30) sont en liaison fluidique l'un avec l'autre par une conduite de recirculation et le récipient de mélange (20) est en liaison fluidique avec les récipients de concentré pour dialyse (10) pour transférer le concentré pour dialyse du récipient de mélange (20) au ou aux récipient(s) de concentré pour dialyse (10).

2. Dispositif selon la revendication 1, **caractérisé en ce qu'**un élément anti-retour est agencé dans l'évacuation et/ou **en ce qu'**un robinet de prélèvement d'échantillon (100) est agencé sur le récipient de concentré pour dialyse (10) et/ou en aval du récipient de concentré pour dialyse dans l'évacuation, sous la forme d'un élément d'arrêt.

3. Dispositif selon l'une des revendications précédentes, **caractérisé en ce que** l'évacuation dépasse dans un liquide sans trajet de chute libre dans sa zone d'extrémité, de telle sorte que le système est exempt d'air.

4. Dispositif selon l'une des revendications précédentes, **caractérisé en ce que** le dispositif présente un appareil de lecture (400) ou un appareil de saisie au moyen duquel le type de concentré, à partir duquel le concentré pour dialyse est fabriqué, peut être lu ou saisi et **en ce qu'**une mémoire est prévue, dans laquelle une corrélation entre le type de concentré et la masse volumique ou la propriété du concentré pour dialyse qui est corrélée avec celle-ci est enregistrée.

5. Dispositif selon la revendication 4, **caractérisé en ce qu'**une unité de comparaison est prévue, qui est conçue pour comparer la masse volumique mesurée ou la propriété qui est corrélée avec celle-ci avec la masse volumique ou la propriété enregistrées dans la mémoire.

6. Dispositif selon la revendication 5, **caractérisé en ce que** l'unité de comparaison est conçue pour émettre un signal dépendant de la comparaison perceptible par l'utilisateur et/ou provoquer l'impression d'une étiquette sur laquelle des données concernant le concentré pour dialyse sont imprimées.

7. Dispositif selon l'une des revendications précédentes, **caractérisé en ce qu'**une commande est prévue, qui active le moyen de mesure (M) seulement après l'écoulement d'un laps de temps défini après l'ouverture de l'élément d'arrêt.

8. Dispositif selon l'une des revendications 4 à 6, **caractérisé en ce qu'**une commande est prévue, qui active le moyen de mesure seulement après l'activation ou l'actionnement de l'appareil de lecture (400) et/ou de l'appareil de saisie.
